# EUROPEAN PATENT APPLICATION

(11) **EP 3 098 220 A1**
(43) Date of publication of application: **30.11.2016**
(21) Application number: 15169687.9
(22) Date of filing: 28.05.2015
(51) Int. Cl.: C07D 471/04, A61K 31/437, A61P 9/10, A61P 11/00, A61P 3/10, A61P 19/08, A61P 25/16

(54) **PROCESS FOR THE PREPARATION OF A NADPH OXIDASE INHIBITOR AND ITS POLYMORPHS AND USES THEREOF**

(71) Applicant: GenKyoTex SA, 1228 Plan-les-Ouates (CH)
(72) Inventor: Hett, Robert, 4132 Muttenz (CH); Pabst, Thomas, 93055 Regensburg (DE)
(74) Representative: reuteler & cie SA

(57) **Abstract**

The present invention is related to a process for the large scale preparation of a nicotinamide adenine dinucleotide phosphate (NADPH) oxidase inhibitor and also relates to polymorphs of that compound, and to methods of isolating a specific polymorph of that compound.

## Description

### Field of the Invention

The present invention relates to a process for the large scale preparation of a nicotinamide adenine dinucleotide phosphate (NADPH) oxidase inhibitor and also relates to polymorphs of that compound, and to methods of isolating a specific polymorph of that compound.

### Background of the Invention

NADPH oxidase (NOX) enzymes exist in seven isoforms and produce reactive oxygen species (ROS) (Leto et al., 2006, Antioxid. Redox Signal, 8(9-10):1549-61*;* Cheng et al., 2001, Gene, 16; 269(1-2):131-40*).* It has been extensively documented that ROS can cause tissue damage and modify biological pathways that are important in a number of pathologies, including metabolic, cardiovascular, pulmonary and neurological diseases *(*Cai et al., 2003, Trends Pharmacol. Sci., 24:471-478*;* Nunomura et al., 2001, J. Neuropathol. Exp. Neurol., 60:759-767*;* Girouard, 2006, J. Appl. Physiol., 100:328-335). For example, in the kidney, NOX4 is the most abundantly expressed isoform and even further upregulated in diabetic nephropathy. The causal role of NOX4 enzymes in various disorders, including diabetic complications, glomerular damage and kidney fibrosis and ostoeclastogenesis is now well recognized (WO 2013/068972). NOX1 is also involved in various disorders including including cardiovascular disorders and in particular hypertension and atherosclerosis, neurodegenerative diseases, liver fibrosis, cancer, in particular in colon cancer, ischemic conditions, in particular ischemic retinopathies and neoplasia and other diabetic co-morbidities (Rynayhossani et al., 2013, J. Bio. Chem., 288(51 ): 36437-50*;* Gianni et al., 2010, ACS Chem. Biol., 5(10):981:93*).*

Therefore, Nox inhibitors have been recently described (WO 2008/113856; WO 2011/036651, Garrido-Urbani, 2011, PLoS ONE, 6(2*),* Wilkinson-Berka et al, 2014, Antioxid. Redox Signal, 20(17):2726-40*)* for addressing treatment needs for those disorders. In particular, selective NOx inhibition is aimed at (WO 2010/035221, WO 2013/068972) and selective inhibitors of both the NOX1 and NOX4 enzymes are of particular interest. Among those, a pyrazolo pyridine derivative, 2-(2-chlorophenyl)-4-[3-(dimethylamino)phenyl]-5-methyl-1H-pyrazolo[4,3-c] pyridine-3,6(2H,5H)-dione of Formula (I): 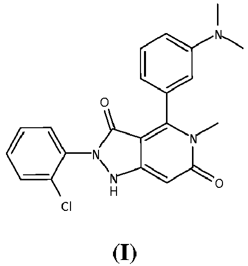 is a pharmaceutically active compound particularly useful for the treatment of diabetic complications, in particular diabetic nephropathy among other NADPH oxidase related disorders such as those described in WO 2010/035221.

Therefore, due to the therapeutic potential of this molecule, there is a need to finding cost-effective processes of preparation which are suitable for large production scales and leading to large quantities of the material in good yield and high purity where the end product would be useful for formulating in view of a commercial use.

### Summary of the Invention

The present invention is directed towards the unexpected findings that the desired product (that is 2-(2-chlorophenyl)-4-[3-(dimethylamino)phenyl]-5-methyl-1H-pyrazolo[4,3-c] pyridine-3,6(2H,5H)-dione) has an improved stability as a free base rather than in the form of salts, in particular a hydrochloride salt, leading to improved properties useful for handling (solubility), quality control (melting point, hydration free) and storage (stability) of the material. Further, the invention is also based on the unexpected findings that such a free base form of said product is capable of existing in at least two different crystalline forms or mixtures thereof, the most stable of which being polymorph form B. The invention further relates to a process for preparation of the polymorph B of said product resulting in the obtaining of polymorph B in an essentially pure form with high yields. This polymorph further presents one advantage to be stable under relative humidity environment.

A first aspect of the invention relates to a crystalline free base form of 2-(2-chlorophenyl)-4-[3-(dimethylamino)phenyl]-5-methyl-1H-pyrazolo [4,3-c]pyridine-3,6(2H,5H)-dione, in particular a crystalline free base form comprising a crystalline polymorph B as characterized by a powder x-ray diffraction pattern as defined herein. A second aspect relates to a pharmaceutical composition comprising a crystalline free base form of 2-(2-chlorophenyl)-4-[3-(dimethylamino)phenyl]-5-methyl-1H-pyrazolo[4,3-c]pyridine-3,6(2H,5H)-dione of the invention and a pharmaceutically acceptable carrier, diluent or excipient.

A third aspect relates to a process for preparing crystalline freebase forms of the compound of Formula (I), in particular form B in an essentially pure form.

A fourth aspect of the invention provides a use of a crystalline free base form of the invention as a medicament.

A fifth aspect relates to a use of a crystalline free base form of the invention for the preparation of a pharmaceutical composition for the treatment and/or prophylaxis of a NADPH oxidase related disorder.

A sixth aspect of the invention relates to a crystalline free base form of the invention for the treatment and/or prophylaxis of a NADPH oxidase related disorder.

A seventh aspect of the invention relates to a method for treating a patient suffering from a NADPH oxidase related disorder, comprising administering an effective amount of a crystalline free base form of a compound according to Formula (I), in a patient in need thereof.

Other features and advantages of the invention will be apparent from the following detailed description.

### Description of the figures

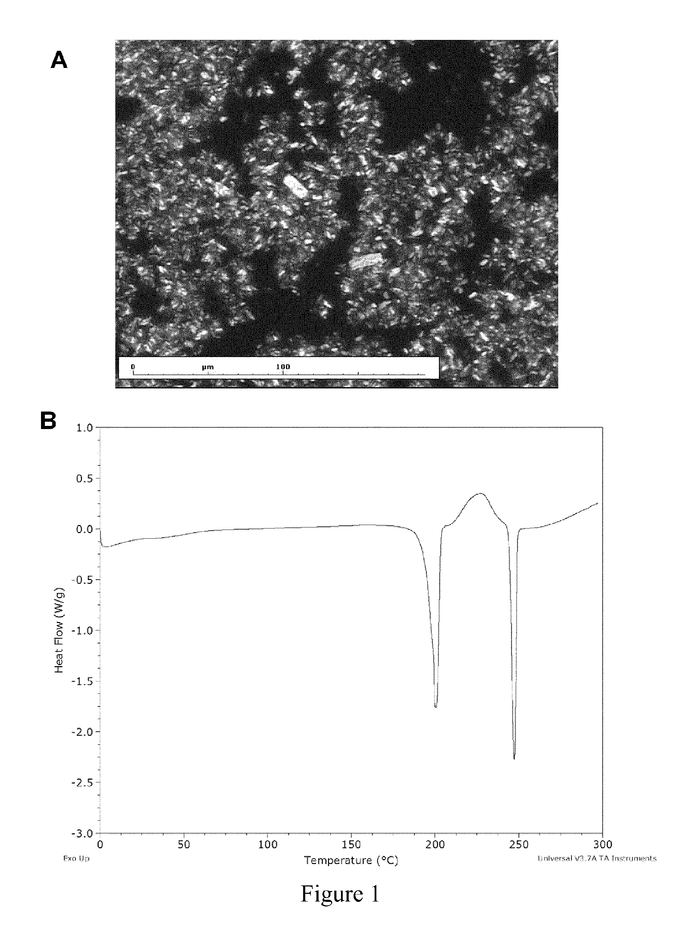

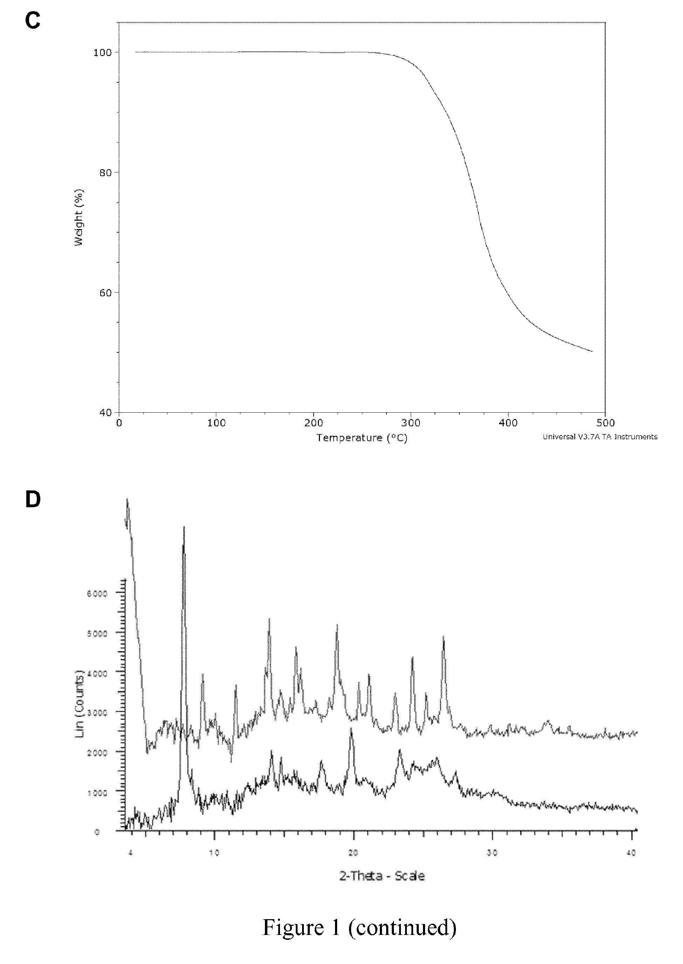

**Figure 1** shows characterization of the free base as obtained under Example 1 as compared to the HCl salt as described in Example 1. **A:** Optical Microscopy image of the free base recrystallized in ethanol/water; **B:** DSC scan of the free base recrystallized in ethanol/water; **C:** Thermogravimetry analysis of the free base recrystallized in ethanol/water; **D:** XRD analysis of the free base recrystallized in ethanol/water (upper spectrum) as compare to the HCl salt (lower spectrum).

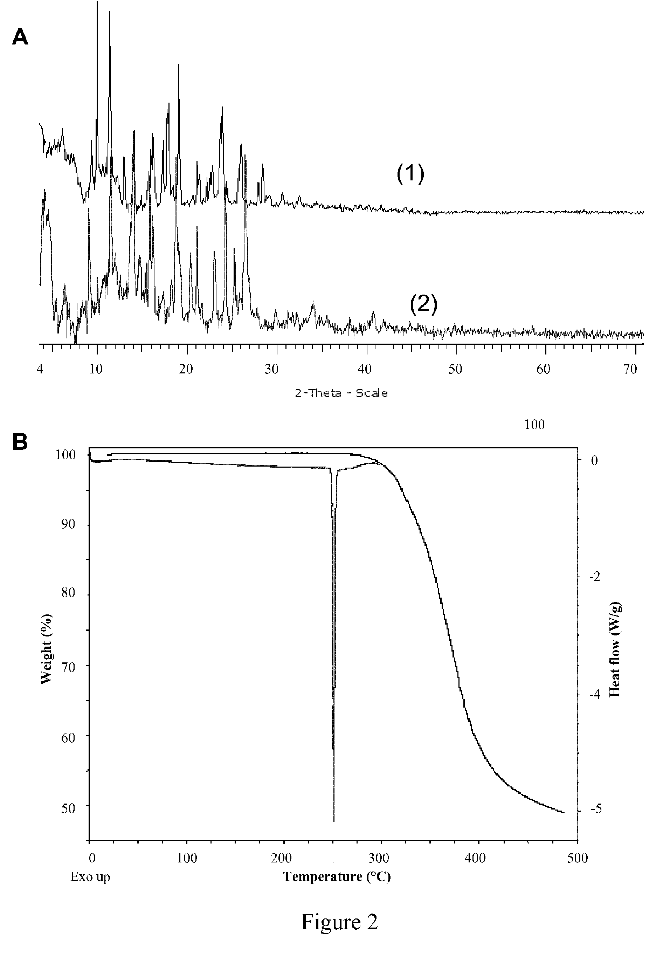

**Figure 2** shows characterization of the polymorph form A and Polymorph B form from Example 1 (Form B) and 2 (Form A) as described in Example 2; **A:** XRD analysis; B: DSC scan and thermogravimetry analysis of Form B.

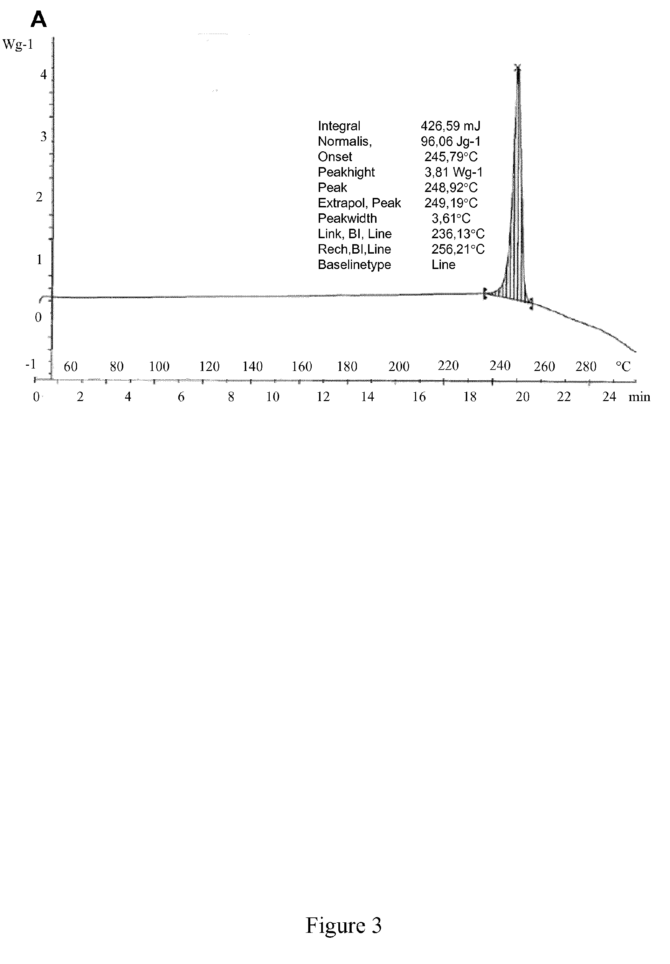

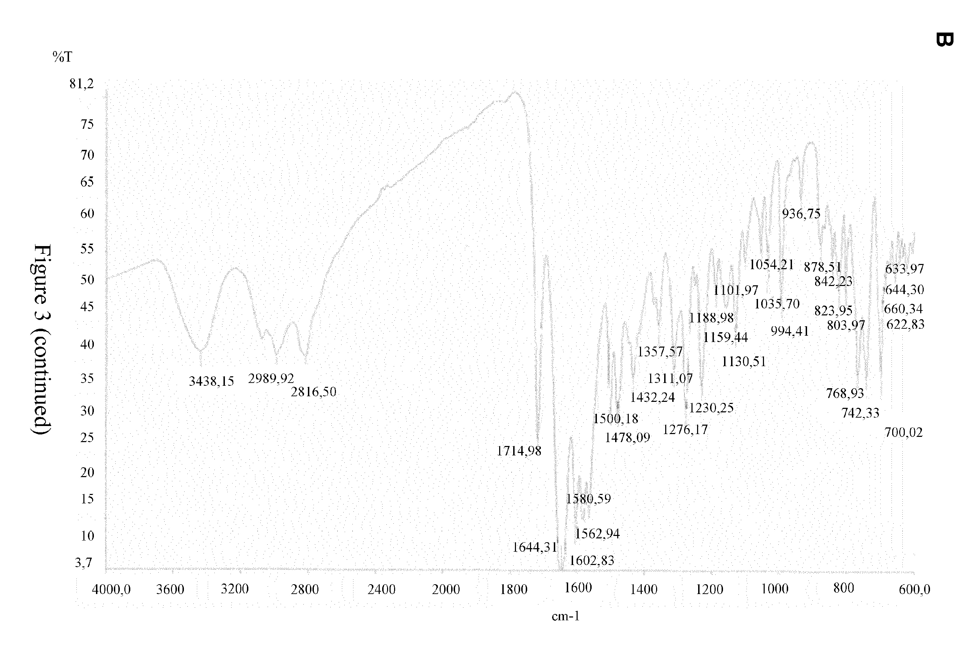

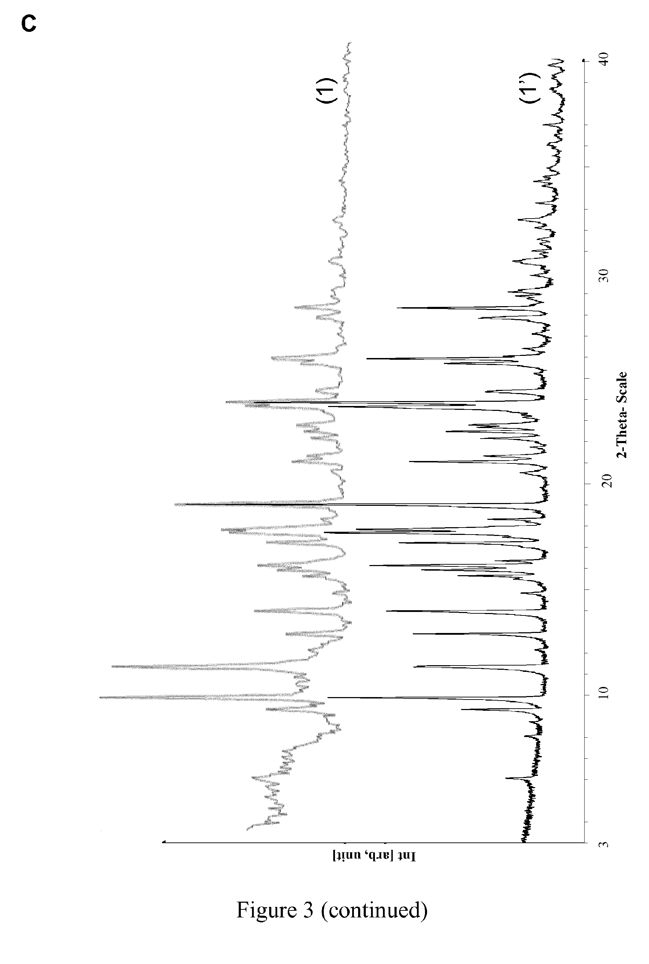

**Figure 3** shows characterization of the polymorph Form B obtained by the process of Example 3 with codistillation of a mixture of methanol and ethyl acetate; **A:** Thermogravimetry analysis; **B:** IR spectrum; C: XRD analysis of the free base form B obtained by the process of the invention (1') as compared to the XRD spectrum of the form B obtained in Example 2(1).

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

The term "polymorph" or "crystalline polymorph" refers to a crystal modification which can be characterized by analytical methods such as e.g. X-ray powder diffraction, IR spectroscopy and Raman spectroscopy.

**Form A:** This polymorph can be produced by crystallizing 2-(2-chlorophenyl)-4-[3-(dimethylamino)phenyl]-5-methyl-1H-pyrazolo[4,3-c] pyridine-3,6(2H,5H)-dione free base form from aqueous alcoholic solvents, for example aqueous ethanol or aqueous isopropanol. Form A has been shown to be characterized by an X-ray pattern as shown on Figure 2A (2).

**Form B:** This polymorph is produced by crystallizing a solution of -(2-chlorophenyl)-4-[3-(dimethylamino)phenyl]-5-methyl-1H-pyrazolo[4,3-c] pyridine-3,6(2H,5H)-dione free base form as described below and in Example 3. The X-ray analysis of the crystals was distinctly different from polymorph A (see Figure 2A (1)). Polymorph B thermodynamically more stable than Form A.

The term "effective amount" as used herein refers to an amount of at least one compound of Formula (I) of the invention or a pharmaceutical formulation thereof according to the invention that elicits a detectable amelioration of the symptoms of the disorders.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "subject" as used herein refers to mammals. For examples, mammals contemplated by the present invention include human, primates, domesticated animals such as cattle, sheep, pigs, horses and the like as well as rodents.

The term "NADPH oxidase related disorders" include any disorder related to any disorders involving NADPH-oxidase-related oxidative damage. This term includes cardiovascular disorders or diseases, respiratory disorders or diseases, infectious disorders or diseases, allergic disorders, traumatisms, diseases or disorders affecting the metabolism, skin diseases, bone disorders, neurodegenerative diseases or disorders, psychotic disorders, kidney diseases or disorders, reproduction disorders or diseases, diseases or disorders affecting the eye and/or the lens, conditions affecting the inner ear, inflammatory disorders or diseases, liver diseases or disorders, pain, cancer, diseases or disorders of the gastrointestinal system, angiogenesis, fibrotic diseases or disorders or the related diseases which require the administration to a mammal in a therapeutic effective dose of a compound expressed by Formula (I) in a sufficient dose to inhibit NADPH oxidase.

The term "cardiovascular disorder or disease" comprises atherosclerosis, especially diseases or disorders associated with endothelial dysfunction including but not limited to hypertension, cardiovascular complications of Type I or Type II diabetes, intimal hyperplasia, coronary heart disease, cerebral, coronary or arterial vasospasm, endothelial dysfunction, heart failure including congestive heart failure, peripheral artery disease, restenosis, trauma caused by a stent, stroke, ischemic attack, vascular complications such as after organ transplantation, myocardial infarction, hypertension, formation of atherosclerotic plaques, platelet aggregation, angina pectoris, aneurysm, aortic dissection, ischemic heart disease, ischemic retinopathies, cardiac hypertrophy, pulmonary embolus, thrombotic events including deep vein thrombosis, injury caused after ischemia by restoration of blood flow or oxygen delivery as in organ transplantation, open heart surgery, angioplasty, hemorrhagic shock, angioplasty of ischemic organs including heart, brain, liver, kidney, retina and bowel.

The term "respiratory disorder or disease" comprises bronchial asthma, bronchitis, allergic rhinitis, adult respiratory syndrome, cystic fibrosis, lung viral infection (influenza), pulmonary hypertension, idiopathic pulmonary fibrosis and chronic obstructive pulmonary diseases (COPD).

The term "infectious disorder or disease" includes a disorder caused by organisms such as bacteria, viruses or parasites. Many organisms live in and on our bodies. It includes but is not limited to infectious diseases of the lung, influenza and other conditions caused by virus infections.

The term "allergic disorder" includes hay fever and asthma.

The term "traumatism" includes polytraumatism.

The term "disease or disorder affecting the metabolism" includes obesity, metabolic syndrome and Type II diabetes.

The term "skin disease" or disorder" includes psoriasis, eczema, scleroderma, xeroderma pigmentosum, skin cancers, melanoma, erythropoietic protoporphyria, discoid lupus erythematosus, solar urticaria, polymorphous light eruption, dermatitis, wound healing and scar formation.

The term "bone disorder" includes osteoporosis, osteoarthritis, osteosclerosis, periodontitis, and hyperparathyroidism.

The term "neurodegenerative disease or disorder" comprises a disease or a state characterized by a central nervous system (CNS) degeneration or alteration, especially at the level of the neurons such as Alzheimer's disease, Parkinson's disease, Huntington's disease, amyotrophic lateral sclerosis, epilepsy and muscular dystrophy. It further comprises neuro-inflammatory and demyelinating states or diseases such as leukoencephalopathies, and leukodystrophies.

The term "demyelinating" is referring to a state or a disease of the CNS comprising the degradation of the myelin around the axons. In the context of the invention, the term demyelinating disease is intended to comprise conditions which comprise a process that demyelinate cells such as multiple sclerosis, progressive multifocal leukoencephalopathy (PML), myelopathies, any neuroinflammatory condition involving autoreactive leukocyte within the CNS, congenital metabolic disorder, a neuropathy with abnormal myelination, drug induced demyelination, radiation induced demyelination, a hereditary demyelinating condition, a prion induced demyelinating condition, encephalitis induced demyelination or a spinal cord injury. Preferably, the condition is multiple sclerosis.

The term "psychotic disorder" includes disorders also known as behavioural disorders or mood disorders and refers to a group of disorders characterized by dramatic changes or extremes of mood which can be for example diagnosed as described in Diagnostic and Statistical Manual of Mental Disorders-4th Edition Text Revision (DMS-IV-TR), American Psychiatric Press, 2000. It includes schizophrenia, schizoaffective disorder, schizophreniform disorder, delusional disorder, psychotic depression, or mania with psychosis.

The term "kidney disease or disorder" includes diabetic nephropathy, renal failure, glomerulonephritis, nephrotoxicity of aminoglycosides and platinum compounds and hyperactive bladder. In a particular embodiment, the term according to the invention includes chronic kidney diseases or disorders.

The term "reproduction disorder or disease" includes erectile dysfunction, fertility disorders, prostatic hypertrophy and benign prostatic hypertrophy.

The term "disease or disorder affecting the eye and/or the lens" includes cataract including diabetic cataract, re-opacification of the lens post cataract surgery, diabetic and other forms of retinopathies like Glaucoma, Aged-related Macular degeneration (AMD), Dry eye syndrome and allergic conjonctivits.

The term "conditions affecting the inner ear" includes presbyacusis, tinnitus, Meniere's disease and other balance problems, utriculolithiasis, vertigo, vestibular migraine, and noise induced hearing loss and drug induced hearing loss (ototoxicity).

The term "inflammatory disorder or disease" means inflammatory bowel disease, sepsis, septic shock, adult respiratory distress syndrome, pancreatitis, shock induced by trauma, bronchial asthma, allergic rhinitis, rheumatoid arthritis, chronic rheumatoid arthritis, arteriosclerosis, intracerebral hemorrhage, cerebral infarction, heart failure, myocardial infarction, psoriasis, cystic fibrosis, stroke, acute bronchitis, chronic bronchitis, acute bronchiolitis, chronic bronchiolitis, osteoarthritis, gout, myelitis, ankylosing spondylitis, Reuter syndrome, psoriatic arthritis, spondylarthritis, juvenile arthritis or juvenile ankylosing spondylitis, reactive arthritis, infectious arthritis or arthritis after infection, gonococcal arthritis, syphilitic arthritis, Lyme disease, arthritis induced by "angiitis syndrome," polyarteritis nodosa, anaphylactic angiitis, Luegenec granulomatosis, rheumatoid polymyalgia, articular cell rheumatism, calcium crystal deposition arthritis, pseudogout, non-arthritic rheumatism, bursitis, tendosynovitis, epicondyle inflammation (tennis elbow), carpal tunnel syndrome, disorders by repetitive use (typing), mixed form of arthritis, neuropathic arthropathy, hemorrhagic arthritis, vascular peliosis, hypertrophic osteoarthropathy, multicentric reticulohistiocytosis, arthritis induced by specific diseases, blood pigmentation, sickle cell disease and other hemoglobin abnormality, hyperlipoproteinemia, dysgammaglobulinemia, hyperparathyroidism, acromegaly, familial Mediterranean fever, Bechet's disease, systemic autoimmune disease erythematosus, multiple sclerosis and Crohn's disease or diseases like relapsing polychondritis, chronic inflammatory bowel diseases (IBD). The term "liver diseases or disorders" include liver fibrosis, alcohol induced fibrosis, steatosis and non alcoholic steatohepatitis.

The term "arthritis" means acute rheumatic arthritis, chronic rheumatoid arthritis, chlamydial arthritis, chronic absorptive arthritis, chylous arthritis, arthritis based on bowel disease, filarial arthritis, gonorrheal arthritis, gouty arthritis, hemophilic arthritis, hypertrophic arthritis, juvenile chronic arthritis, Lyme arthritis, neonatal foal arthritis, nodular arthritis, ochronotic arthritis, psoriatic arthritis or suppurative arthritis, or the related diseases which require the administration to a mammal in a therapeutic effective dose of a compound expressed by Formula (I) in a sufficient dose to inhibit NADPH oxidase.

The term "pain" includes hyperalgesia associated with inflammatory pain and neurogenic pain.

The term "cancer" means carcinoma (e.g., fibrosarcoma, myxosarcoma, liposarcoma, chondrosarcoma, osteogenic sarcoma, chordoma, angiosarcoma, endothelium sarcoma, lymphangiosarcoma, lymphangioendothelioma, periosteoma, mesothelioma, Ewing's tumor, leiomyosarcoma, rhabdomyosarcoma, colon carcinoma, pancreatic cancer, breast cancer, ovarian cancer, renal cancer, prostatic carcinoma, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, hepatocellular carcinoma, cholangiocarcinoma, choriocarcinoma, seminoma, embryonal carcinoma, Wilms' tumor, cervical cancer, orchioncus, lung cancer, small-cell lung cancer, lung adenocarcinoma, bladder cancer or epithelial cancer, melanoma), neoplasia or the related diseases which require the administration to a mammal in a therapeutic effective dose of a compound expressed by the Formula (I) in a sufficient dose to inhibit NADPH oxidase. In particular, disorders induced by the toxicity of some drugs such as cancer therapies (e.g. Doxorubicin).

The term "disease or disorders of the gastrointestinal system", includes gastric mucosa disorders ischemic bowel disease management, enteritis/colitis/Crohn's Disease, cancer chemotherapy, or neutropenia.

The term "angiogenesis" includes sprouting angiogenesis, intussusceptive angiogenesis, vasculogenesis, arteriogenesis and lymphangiogenesis. Angiogenesis is the formation of new blood vessels from pre-existing capillaries or post-capillary venules and occurs in pathological conditions such as cancers, arthritis and inflammation. A large variety of tissues, or organs comprised of organized tissues, can support angiogenesis in disease conditions including skin, muscle, gut, connective tissue, joints, bones and the like tissue in which blood vessels can invade upon angiogenic stimuli. As used herein, the term "angiogenesis-dependent condition" is intended to mean a condition where the process of angiogenesis or vasculogenesis sustains or augments a pathological condition. Vasculogenesis results from the formation of new blood vessels arising from angioblasts which are endothelial cell precursors. Both processes result in new blood vessel formation and are included in the meaning of the term angiogenesis-dependent conditions. Similarly, the term "angiogenesis" as used herein is intended to include de novo formation of vessels such as those arising from vasculogenesis as well as those arising from branching and sprouting of existing vessels, capillaries and venules.

The term "fibrotic disease or disorder" refers to diseases or disorders characterized by the development of excess fibrous connective tissue as a reparative response to injury or damage and includes pulmonary fibrosis, kidney fibrosis, liver fibrosis, retroperitoneal fibrosis and heart fibrosis.

### Compound according to the Invention and polymorphs thereof

In one embodiment, the invention provides a crystalline free base form of 2-(2-chlorophenyl)-4-[3-(dimethylamino)phenyl]-5-methyl-1H-pyrazolo [4,3-c]pyridine-3,6(2H,5H)-dione which is characterized by an X-ray powder diffraction pattern having characteristic peaks expressed in angle 2-theta at approximately (Form B): 9.9± 0.2°, 11.3± 0.2°, 17.7± 0.2°, 19.0± 0.2° and 23.9± 0.2°.

According to a further embodiment, the invention provides a crystalline free base form of 2-(2-chlorophenyl)-4-[3-(dimethylamino)phenyl]-5-methyl-1H-pyrazolo[4,3-c] pyridine-3,6(2H,5H)-dione which is characterized by an X-ray powder diffraction pattern having characteristic peaks as described in Table 1 below:

**Table 1**

| **Position [°2-theta]** | **Relative Intensity [%]** |
|---|---|
| 6.1 | 40 |
| 9.3 | 35 |
| 9.9 | 100 |
| 10.5 | 23 |
| 10.9 | 24 |
| 11.3 | 95 |
| 12.1 | 18 |
| 12.9 | 27 |
| 14.0 | 39 |
| 14.9 | 9 |
| 15.7 | 20 |
| 16.2 | 38 |
| 17.2 | 35 |
| 17.7 | 49 |
| 18.3 | 13 |
| 19.0 | 70 |
| 20.6 | 9 |
| 21.1 | 25 |
| 21.4 | 19 |
| 22.2 | 17 |
| 22.5 | 20 |
| 22.8 | 23 |
| 23.7 | 43 |
| 23.9 | 50 |
| 24.4 | 15 |
| 25.7 | 21 |
| 25.9 | 33 |
| 27.9 | 15 |
| 28.4 | 23 |
| 28.9 | 10 |
| 29.2 | 10 |
| 30.6 | 10 |

In another embodiment, is provided a crystalline polymorph of the invention (Form A), characterized by X-ray powder diffraction pattern having characteristic peaks expressed in angle 2-theta at approximately: 9.1 ± 0.2°, 11.4 ± 0.2°, 13.9 ± 0.2°, 15.8 ± 0.2°, 16.2 ± 0.2°, 18.7 ± 0.2°, 24.2 ± 0.2° and 26.5 ± 0.2°.

According to a further embodiment, the invention provides a crystalline free base form of 2-(2-chlorophenyl)-4-[3-(dimethylamino)phenyl]-5-methyl-1H-pyrazolo[4,3-c]pyridine-3,6(2H,5H)-dione which is characterized by an X-ray powder diffraction pattern having characteristic peaks as described in Table 2 below:

**Table 2**

| **Position [°2-theta]** | **Relative Intensity [%]** |
|---|---|
| 5.3 | 21 |
| 6.2 | 28 |
| 7.7 | 19 |
| 8.2 | 20 |
| 9.1 | 64 |
| 9.9 | 31 |
| 10.6 | 32 |
| 11.0 | 33 |
| 11.4 | 92 |
| 11.9 | 42 |
| 13.9 | 100 |
| 14,6 | 42 |
| 15.4 | 39 |
| 15.8 | 79 |
| 16.2 | 60 |
| 17.3 | 25 |
| 18.2 | 30 |
| 18.7 | 88 |
| 20.4 | 43 |
| 21.1 | 55 |
| 21.6 | 19 |
| 23.0 | 43 |
| 24.2 | 75 |
| 25.2 | 45 |
| 26.5 | 89 |
| 29.9 | 16 |
| 31.2 | 16 |
| 34.2 | 17 |
| 37.8 | 9 |
| 38.2 | 12 |
| 40.7 | 15 |

In a further embodiment, is provided a crystalline polymorph of the invention, characterized by the X-ray powder diffraction pattern spectrum shown in Figure 3C. In a further embodiment, is provided a crystalline polymorph of the invention, characterized by the IR absorption spectrum shown in Figure 3B.

In a further embodiment, is provided a crystalline polymorph of the invention which is characterized by an IR absorption spectrum having characteristic peaks expressed in cm-1 at approximately 3438 cm-1, 2989 cm-1, 2817 cm-1, 1715 cm-1 1644 cm-1 1602 cm-1 1581 cm-1 1563 cm-1 1500 cm-1 1478 cm-1 1432 cm-1 1358 cm-1 1311 cm-1 1276 cm-1 1230 cm-1 1130 cm-1 994 cm-1, 768 cm-1, 742 cm-1, and 700 cm-1.

In a further embodiment, is provided a crystalline polymorph of the invention, characterized by the IR absorption spectrum shown in Figure 3B.

In another embodiment, a crystalline free base form of 2-(2-chlorophenyl)-4-[3-(dimethylamino)phenyl]-5-methyl-1H-pyrazolo[4,3-c]pyridine-3,6(2H,5H)-dione according to the invention which comprises polymorph B in essentially pure form or a mixture thereof with polymorph A, and polymorph B presents a thermodynamically more stable form of 2-(2-chlorophenyl)-4-[3-(dimethylamino)phenyl]-5-methyl-1H-pyrazolo[4,3-c]pyridine-3,6(2H,5H)-dione which has enhanced physical and chemical stability towards heat and humidity, when compared to the free base in amorphous state or the compound in salt form.

### Preparation of polymorphs of the free base of the invention

In a further embodiment, is provided a process for the preparation of a crystalline free base according to the invention comprising the steps of:
(i) Providing a mixture of an aqueous solution of the 2-(2-chlorophenyl)-4-[3-(dimethylamino)phenyl]-5-methyl-1-H-pyrazolo[4,3-c]pyridine-3,6(2H,5H)-dione free base in an apolar solvent or a mixture thereof such as dichloromethane (DCM), toluene, methyl t-butyl ether (MTBE), methyl ethyl ketone (MEK), methyl isobutyl ketone (MIBK), Benzene, diethylether, ethylacetate, propylacetate, isopropyl acetate, tetrahydrofurane (THF), nButylacetate, hexane and mixtures thereof;
(ii) Adding a polar solvent (such as methanol, ethanol, isopropanol, butanol, isobutanol, acetic acid, acetonitrile, N, N-dimethylformamide) to the mixture;
(iii) Concentrating the mixture *in vacuo* under stirring at a temperature from about 10-50°C, for example from about 20 to about 25°C;
(iv) isolating the solid product.

According to a particularly advantageous embodiment, the invention provides a process for the preparation of a crystalline free base according to the invention, wherein steps from (ii) and to (iii) further comprise the following steps:
(iia) Adding a polar solvent (such as methanol, methanol, ethanol, isopropanol, butanol, isobutanol) to the mixture;
(iiia) concentrating the mixture *in vacuo* under stirring at a temperature from about 10-50°C, for example from about 20 to about 25°C;
(iib) progressively adding a non-polar solvent (such as ethyl acetate, propyl acetate, isopropyl acetate, toluene, methyl t-butyl ether (MTBE), methyl ethyl ketone (MEK), methyl isobutyl ketone (MIBK), Benzene, diethylether, tetrahydrofurane (THF), nButylacetate and mixtures thereof to the mixture during the concentration step for replacing progressively the polar solvent that is evaporating and repeating steps (iiia) to (iib) until the the polar solvent is essentially removed from the mixture, for example that its content is not higher than 5 to 10% vol., typically less than about 5% in vol.;
(iiib) completing the final concentration step.

According to a particular embodiment, the pH of the solution under step (i) is adjusted in a pH range of 5-7, in particular between about 5.5 and about 6.2.

According to another particular embodiment, during the concentration step (iii), a polar is further added at least once.

According to another particular embodiment, the concentration step (iii) last typically between about 12 hours and about 48 hours.

According to another particular embodiment, the isolation step (iv) comprises a step of filtering the resulting mixture.

According to another particular embodiment, the isolation step comprises a step of drying the resulting product.

According to another particular embodiment, the isolation step comprises a step of micronizing the dried the resulting product.

### Compositions

The invention provides pharmaceutical or therapeutic agents as compositions and methods for treating a patient, preferably a mammalian patient, and most preferably a human patient who is suffering from a NADPH related disorder.

Pharmaceutical compositions of the invention can contain one or more compound of Formula (I) in any form described herein. Compositions of this invention may further comprise one or more pharmaceutically acceptable additional ingredient(s), such as alum, solubilizers, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as powder in sachets, tablets or filled capsules, or liquids such as solutions, suspensions, emulsions, elixirs, nasal spray, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably oral, sublingual, nasal and subcutaneous.

Compositions of this invention may also be liquid formulations, including, but not limited to, aqueous or oily suspensions, solutions, emulsions, syrups, spray and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in The Science and Practice of Pharmacy (Remington: The Science & Practice of Pharmacy), 22nd Edition, 2012, Lloyd, Ed. Allen, Pharmaceutical Press*,* which is incorporated herein by reference.

Solid compositions of this invention may be in the form of powder in sachets, tablets or lozenges formulated in a conventional manner. For example, sachets, tablets and capsules for oral or sublingual administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

The compounds of this invention can also be administered in sustained release forms or from sustained release drug delivery systems. A description of representative sustained release materials can also be found in the incorporated materials in *Remington's Pharmaceutical Sciences.*

The invention also relates to a crystalline freebase of the invention in a micronized form; and to pharmaceutical compositions comprising a pharmaceutically acceptable carrier and a micronized crystalline freebase of the invention.

### Mode of administration

Compositions of this invention may be administered in any manner, including, but not limited to, orally, parenterally, sublingually, via buccal administration, nasally, or combinations thereof. Parenteral administration includes, but is not limited to subcutaneous and intramuscular. The compositions of this invention may also be administered in the form of an implant, which allows slow release of the compositions as well as a slow controlled i.v. infusion. In a particular embodiment, one or more the crystalline polymorph of the invention is administered orally.

The dosage administered, as single or multiple doses, to an individual will vary depending upon a variety of factors, including pharmacokinetic properties, patient conditions and characteristics (age, body weight, health, body size), extent of symptoms, frequency of treatment and the effect desired.

### Combination

According to one embodiment of the invention, a crystalline polymorph of the invention according to the invention and pharmaceutical formulations thereof is to be administered in combination with one or more other therapeutic agents (e.g. multiple drug regimens).

Accordingly, in one embodiment, the invention relates to a pharmaceutical composition comprising (a) a pharmaceutically acceptable carrier and a therapeutically effective amount of a crystalline freebase of the invention; and (b) a therapeutically effective amount of an agent selected from an anti-inflammatory agent, a phosphodiesterase-5 inhibitor such as sildenafil or sildenafil citrate; or a combination thereof; wherein the crystalline freebase and the agent are formulated together or separately. When the agent is formulated separately, a pharmaceutically acceptable carrier may be included. Typically, the crystalline freebase of the invention and the agent will be present in therapeutically effective amounts.

The invention encompasses the administration of a crystalline polymorph of the invention or a pharmaceutical formulation thereof, wherein a crystalline polymorph of the invention or a pharmaceutical formulation thereof is administered to an individual prior to, or simultaneously with a further therapeutic agent, for example or concomitantly through the same formulation or different composition(s) and by the same or different route(s) of administration.

### Patients

In an embodiment, patients according to the invention are patients suffering from a cardiovascular disorder or disease, in particular of hypertension, atherosclerosis and ischemic conditions.

In another embodiment, patients according to the invention are patients suffering from a respiratory disorder or disease.

In another embodiment, patients according to the invention are patients suffering from a disease or disorder affecting the metabolism, in particular diabetic disorders.

In another embodiment, patients according to the invention are patients suffering from a skin disorder.

In another embodiment, patients according to the invention are patients suffering from a bone disorder.

In another embodiment, patients according to the invention are patients suffering from a neuroinflammatory disorder and/or a neurodegenerative disorder, in particular Parkinson's disease.

In another embodiment, patients according to the invention are patients suffering from a kidney disease.

In another embodiment, patients according to the invention are patients suffering from a reproduction disorder.

In another embodiment, patients according to the invention are patients suffering from a disease or disorder affecting the eye and/or the lens and/or a condition affecting the inner ear.

In another embodiment, patients according to the invention are patients suffering from an inflammatory disorder or disease.

In another embodiment, patients according to the invention are patients suffering from a liver disease.

In another embodiment, patients according to the invention are patients suffering from pain, such as inflammatory pain.

In another embodiment, patients according to the invention are patients suffering from a cancer, in particular colon cancer.

In another embodiment, patients according to the invention are patients suffering from a fibrotic disorder, in particular liver fibrosis.

In another embodiment, patients according to the invention are patients suffering from a psychotic disorder.

In another embodiment, patients according to the invention are patients suffering from an infectious disease, in particular a viral lung infection or influenza.

In another embodiment, patients according to the invention are suffering from angiogenesis or an angiogenesis-dependent condition.

In another embodiment, patients according to the invention are patients suffering from allergic disorders.

In another embodiment, patients according to the invention are patients suffering from traumatisms.

In another embodiment, patients according to the invention are patients suffering from septic, hemorrhagic and anaphylactic shock.

In another embodiment, patients according to the invention are patients suffering from a disease or disorders of the gastrointestinal system.

### Use according to the invention

In another particular embodiment, is provided a crystalline free base form of Formula (I) according of the invention for use as a medicament.

In another particular embodiment, is provided a crystalline free base form of Formula (I) according of the invention for use in the treatment of a NADPH related disorder, in particular diabetic nephropathy, atherosclerosis, idiopathic pulmonary fibrosis, liver fibrosis and angiogenesis.

Additionally, the invention relates to use of a crystalline freebase of the invention for the manufacture of a medicament, especially for the manufacture of a medicament for the treatment of a NADPH related disorder, in particular diabetic nephropathy, atherosclerosis, idiopathic pulmonary fibrosis, liver fibrosis and angiogenesis in a mammal.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

The crystalline free base forms of the invention were characterized by the following experiments.

**The following abbreviations refer respectively to the definitions below:**
**FTIR** (Fourier-Transform Infrared Red).

### Example 1: Comparison of the salt form and the free base form

### HCl salt

2-(2-chlorophenyl)-4-[3-(dimethylamino)phenyl]-5-methyl-1H-pyrazolo [4,3-c] pyridine-3,6(2H,5H)-dione was prepared as an HCl salt as follows:
The free base is initially solubilized in ethanol. An appropriate volume of a HCl 1N solution (in water) is added to a certain volume of the freebase solution in ethanol so that the free base and its counter ion are in an equimolar ratio. The mixture is stirred at 50°C for a few minutes and then the solvents are removed under *vacuum*.
The film (free base-HC1, in an equimolar ratio) is solubilized with a mixture of 3 solvents: ethyl acetate (about 1 mL for 100 mg free base), ethanol (about 375 µL for 100 mg free base) and water (about 125µL for 100 mg free base). The mixture is stirred at 65°C and becomes clear.
The sample is then allowed to cool down in an ice bath without stirring for several hours. Crystallization occurs. The supernatant is removed from the flask. The powder is dried under dynamic vacuum over a few minutes at 60°C.

The HC1 salt form was also tested for its ability to crystallize in different solvents such as methanol, dichloromethane by addition of concentrated HC1 and also non-aqueous HC1 in isopropanol. In all experiments, no crystallization was observed.

### Free base form

2-(2-chlorophenyl)-4-[3-(dimethylamino)phenyl]-5-methyl-1H-pyrazolo [4,3-c] pyridine-3,6(2H,5H)-dione was prepared as a free base as described in WO 2010/035221.

The free base was then tested for its ability to crystallize in a mixed water /alcohol (ethanol) (1v/1v) and in mixed water/isopropanol (1v/1v) media as follows:
Recrystallization was achieved by (successive) heating and cooling steps (dissolution by heating and recrystallization upon cooling the supersaturated solution). The amount of free base to be recrystallized is weighed in a glass vessel. The required volume of recrystallization medium is added onto the powder and the resulting suspension is then stirred while being heated up to a temperature close to the tested organic solvent reflux.
After complete dissolution of the powder, the mixture is kept at this temperature for 10 to 20 minutes. It is then filtered and allowed to cool down to room temperature.

As an example, 1 mL of ethanol/water (1v/1v) is utilized to re-crystallize (as described above) 50 mg of 2-(2-chlorophenyl)-4-[3-(dimethylamino)phenyl]-5-methyl-1H-pyrazolo[4,3-c] pyridine-3,6(2H,5H)-dione free base (water can then be added to wash the crystals and residual ethanol before drying). Seeding can be utilized to successfully initiate the crystallization of larger crystals. In order to improve the size of the crystals, a sample can been re-dissolved by a second heating (after a first crystallization) and recrystallized again. In ethanol/water (1v/1v), 2-(2-chlorophenyl)-4-[3-(dimethylamino)phenyl]-5-methyl-1H-pyrazolo[4,3-c] pyridine-3,6(2H,5H)-dione free base crystallization is obtained in a satisfactory manner without requiring to carefully manage the temperature ramp (crystallization is obtained by allowing the supersaturated solution to cool down to room temperature).

As another example, 95 mg of the free base are recrystallized in 2 mL of an isopropanol /water (1 v/ 1v) mixture, by dissolution after heating the suspension at 70°C, filtering it on a 0.45µm porosity disposable filter, and letting the solution to cool down to room temperature. By doing so, crystallization occurs in the next 2 to 3 hours. The supernatant is then removed and the recrystallized sample washed with water before being dried under dynamic vacuum.

Both the HC1 salt and the free base were compared for their solubility, ability to crystallize and their hygroscopic properties in the assays as described below.

### Solubility assessment

The solubility of the HC1 salt and of the free base was compared in a pH1 HC1/KC1 buffer and in a pH 7.4 phosphate buffer. For each medium, the saturation of the medium is obtained by adding an excess of active ingredient to a given volume of test medium. The suspension is stirred over 24 hours at 20°C, protected from light. The supernatant is then isolated by centrifugation, diluted in a solvent mixture allowing its injection into the chromatographic system. The compound concentration in solution for each medium is determined by external standardization and HPLC under the conditions detailed in Table3 below. Solubility was tested over a range from 1 µg/mL up to 1 mg/mL.

**Table 3**

| | | | |
|---|---|---|---|
| Column | Symmetryshield | | |
| | 50 mm x 2.1 mm - dp = 3.5 µm | | |
| Mobile phase | Solvent A: H₂O/TFA0.05% | | |
| | Solvent B: CH₃CN/TFA0.05% | | |
| | Time (min) | A % | B % |
| | 0 | 95 | 5 |
| | 0.3 | 95 | 5 |
| | 2.5 | 10 | 90 |
| | 3.5 | 10 | 90 |
| | 3.6 | 95 | 5 |
| | 4.0 | 95 | 5 |
| Flow | 1 mL/min | | |
| Column temperature | Ambient temperature | | |
| Detection | UV-DAD | | |
| Solutions test | Dilution with H₂O/CH₃CN 50/50 (v/v) or H₂O/CH₃CN 80/20 (v/v) | | |
| Injection volume | 10 µL | | |
| Injector temperature | 20°C | | |

### Optical microscopy (OM)

Analysis of the dried samples after the crystallization process was carried out by optical microscopy is performed on a LEICA DMIRB microscope equipped with a digital camera and a motorized stage. Acquisition of microscopy patterns is performed with an image analysis station. After carrying out the crystallization protocol and the obtained powder is dried under dynamic vacuum over few minutes at 70°C and then further dried at 40°C for a few hours, a few milligrams of the dried powder are dropped onto a microscopic glass and analysed. Various images are recorded with transmitted light and then polarized light in order to characterize the crystallinity of the products.

The preliminary characterization of the HCl salt indicated that it is amorphous or poorly crystalline as shown by optical microscopy, whereas, the preliminary characterization of the free base samples obtained as described above shows that the material is crystalline as shown on Figure 1A.

### Differential scanning calorimetry (DSC)

Differential scanning calorimeter (DSC) analysis is performed on a Q1000 TA Instruments analyzer. The instrumental operating conditions for DSC profile acquisition are described in Table 4.

**Table 4**

| | | |
|---|---|---|
| Heater ramp (°C/ min) | | 10 |
| Final temperature (°C) | | 300 |
| Carrier gas | - nitrogen | Messer, « qualité Azote 5.0 » |
| | - flow rate (mL/min) | 50 |

The preliminary characterization of the HC1 salt obtained as described above confirmed that it is poorly crystalline or amorphous.

The preliminary characterization of the free base samples obtained as described above shows that the material is crystalline with a melting temperature above 190°C as shown on Figure 1B and in Table 5 below for the free base batch recrystallized in ethanol/water:

**Table 5**

| **Event** | **Transition** | **Temperature (°C)** | | **Enthalpy transition** |
|---|---|---|---|---|
| | | Onset | Peak | (J/g) |
| 1 | endotherm | 198 | 200 | 69 |
| 2 | exotherm | 211 | 227 | 31 |
| 3 | endotherm | 244 | 247 | 43 |

Several events are present in the DSC patterns of the three produced samples: endotherm, exotherm and endotherm. In order to assess the hypothesis of the degradation in the melt/recrystallization as a possible origin for the second endotherm, about 5 mg of the sample recrystallized in ethanol/water is introduced into the DSC and heated to 240°C (before the second endotherm).

This sample is then collected from the DSC pan and solubilized in a solvent mixture to be injected into the chromatographic system. The reverse phase chromatographic profile (retention time, UV spectrum) of this heated sample is compared to the one of an unstressed sample (sample recrystallized in ethanol/water). The obtained chromatographic profiles of both samples (unstressed sample and "melted/recrystallized" sample) are very similar and therefore the degradation hypothesis could be discarded.

The DSC profile is therefore due to pseudo polymorphism (hydrate form) or polymorphism of the free base. Therefore, the sample was further tested by TGA in order to discard the hypothesis of a solvated or a hydrate form.

### Thermogravimetric analysts coupled to infrared spectroscopy (TGA-FTIR)

Thermogravimetric analysis is performed on a TA Instruments TGA Hi-Res 2950 apparatus equipped with an evolved gas analysis furnace. A ThermoNicolet Nexus FTIR bench, equipped with FTIR cell gas analysis, is coupled to TGA apparatus via a transfer line.

Upon heating of the sample of the free base batch recrystallized in ethanol/water, no mass loss is detected before the main thermal decomposition step that occurs with an onset temperature of 299°C, i.e. before or during the melting transition. Hence, the free base physical form present in this sample corresponds to a non-solvated crystalline phase.

### Hygroscopicity assessment by Dynamic water vapor sorption/desorption analysis (DVS)

The dynamic vapor sorption (DVS) analysis with water is performed on a DVS-Intrinsic incubator from SMS Ltd, equipped with DVS-Intrinsic Control Software 1.0. A sample weight of about 8 to 10mg, placed in an aluminum pan holder, is submitted to a full cycle analysis (sorption followed by desorption) in a dm/dt mode according to the conditions described in Table 6 below.

**Table 6**

| **Temperature** | **25°C** |
|---|---|
| Carrier gas and rate | dried and filtered air at 100ml.min⁻¹ |
| Mode and criterion | dm/dt ≤ 0.002%.min⁻¹ |
| Humidity range | 0 to 95%RH |
| RH step | 5% |
| Minimum step time | 10 minutes |
| Maximum step time | 360 minutes |

The sample obtained after crystallization protocol is pre-dried under a stream of dry filtered air until a stable weight is obtained. Next, relative humidity is increased by 5% increments. For each step, the sample mass is allowed to increase until equilibrium is reached (dm/dt criterion), and then a new relative humidity rise occurs. Relative humidity is ramped up to 90%. After equilibration at this point, desorption begins in a similar stepwise fashion, with sample weight again allowed to stabilize after each incremental humidity decrease. For each segment of the method described above, the recording of the sample mass allows describing of the whole sorption/desorption behavior versus relative humidity.

The sample mass uptake of the free base is 0.2% at 60% or relative humidity and 0.3 at 95% of relative humidity.

Based on the above results, it is concluded that the HC1 salt is rather amorphous or poorly crystalline as opposed to the free base that was characterized as non-hygroscopic (commonly used criteria: 2% maximum uptake at 60% relative humidity) and crystalline.

### Example 2: Characterization of polymorph Form A and polymorph Form B the free base form

Two batches of the free base batch recrystallized in ethanol/water (sample 2 and sample 1 which was obtained after subjecting sample 1 to a temperature around 240°C) were characterized by X-ray powder diffraction (XRPD) as described below:

### X-ray powder diffraction (XRPD)

XRPD analysis is performed on a Bruker - AXS D8 Advance diffractometer, using a copper anti-cathode, a mono-crystalline silicon sample holder and a position sensitive detector. Instrument operating conditions for X-rays pattern acquisition are described in Table 7 below. Powder sample obtained crystallisation is dispersed on the silicon sample holder in a way to avoid preferred orientation (not randomly oriented crystals) and to ensure planarity of the specimen surface.

**Table 7**

| | | | |
|---|---|---|---|
| Temperature | | | Ambient |
| Atmosphere | | | Ambient |
| X-rays generator | - voltage (kV) | | 40 |
| | - intensity (mA) | | 40 |
| X-rays source | - target | | Cu |
| | - emission radiation | Kλ1 (nm) | 0.15406 |
| | | Kλ2 (nm) | 0.15444 |
| | | ratio Kλ2 / Kλ1 | 0.5 |
| | - Kβ filter radiation | | Nickel |
| Slit (mm) | - anti-divergence | | 0,6 |
| Goniometer | - angular sector analyzed (° for 2θ) | | 4-70 |
| | - step size (° for 2θ) | | 0.069 |
| Rotation speed for sample holder (rpm) | | | 30 |
| Detection | - angular opening (°) | | 8 |
| | - step time for measuring diffracted intensity (s) | | 6 |

The diffraction profile of the free base from sample 1 clearly evidences an organic material of good crystallinity with numerous, intense and well-defined diffraction peaks between 10 and 30°. No amorphous phase is detected in the tested operating conditions. A different diffraction peak profile is obtained for sample 2 as shown in Figure 2 A where it can be seen that differences on diffraction peak positions are detected between the two batches patterns. The crystalline phase present in batch sample 1 will be called crystal form "B" and the one present in batch sample 2 will be called crystal form "A".

The polymorph form A is characterized by a powder X-ray diffraction pattern (PXRD) which includes main peaks a 2-Theta angles 9.1 ± 0.2°, 11.4 ± 0.2°, 13.9 ± 0.2°, 15.8 ± 0.2°, 16.2 ± 0.2°, 18.7 ± 0.2°, 24.2 ± 0.2° and 26.5 ± 0.2°., as shown in Table 8 below.

**Table 8**

| Angle | Intensity | |
|---|---|---|
| 2-Theta (°) | Cps | % |
| 5.3 | 631 | 21 |
| 6.2 | 841 | 28 |
| 7.7 | 556 | 19 |
| 8.2 | 596 | 20 |
| 9.1 | 1911 | 64 |
| 9.9 | 940 | 31 |
| 10.6 | 953 | 32 |
| 11.0 | 992 | 33 |
| 11.4 | 2761 | 92 |
| 11.9 | 1267 | 42 |
| 13.9 | 2999 | 100 |
| 14,6 | 1247 | 42 |
| 15.4 | 1167 | 39 |
| 15.8 | 2375 | 79 |
| 16.2 | 1808 | 60 |
| 17.3 | 735 | 25 |
| 18.2 | 896 | 30 |
| 18.7 | 2630 | 88 |
| 20.4 | 1274 | 43 |
| 21.1 | 1642 | 55 |
| 21.6 | 567 | 19 |
| 23.0 | 1300 | 43 |
| 24.2 | 2244 | 75 |
| 25.2 | 1344 | 45 |
| 26.5 | 2655 | 89 |
| 29.9 | 483 | 16 |
| 31.2 | 468 | 16 |
| 34.2 | 522 | 17 |
| 37.8 | 278 | 9 |
| 38.2 | 362 | 12 |
| 40.7 | 461 | 15 |

The polymorph form B is characterized by a powder X-ray diffraction pattern (PXRD) which includes main peaks a 2-Theta 9.9 ± 0.2°, 11.3 ± 0.2°, 17.7 ± 0.2°, 19.0 ± 0.2° and 23.9 ± 0.2°, as shown in Table 9 below.

**Table 9**

| Angle | Intensity | |
|---|---|---|
| 2-Theta (°) | Cps | % |
| 6.1 | 2901 | 40 |
| 9.3 | 2499 | 35 |
| 9.9 | 7227 | 100 |
| 10.5 | 1658 | 23 |
| 10.9 | 1710 | 24 |
| 11.3 | 6885 | 95 |
| 12.1 | 1309 | 18 |
| 12.9 | 1950 | 27 |
| 14.0 | 2838 | 39 |
| 14.9 | 624 | 9 |
| 15.7 | 1467 | 20 |
| 16.2 | 2737 | 38 |
| 17.2 | 2497 | 35 |
| 17.7 | 3547 | 49 |
| 18.3 | 947 | 13 |
| 19.0 | 5088 | 70 |
| 20.6 | 656 | 9 |
| 21.1 | 1772 | 25 |
| 21.4 | 1350 | 19 |
| 22.2 | 1233 | 17 |
| 22.5 | 1418 | 20 |
| 22.8 | 1649 | 23 |
| 23.7 | 3097 | 43 |
| 23.9 | 3632 | 50 |
| 24.4 | 1096 | 15 |
| 25.7 | 1521 | 21 |
| 25.9 | 2358 | 33 |
| 27.9 | 1066 | 15 |
| 28.4 | 1683 | 23 |
| 28.9 | 707 | 10 |
| 29.2 | 690 | 10 |
| 30.6 | 731 | 10 |
| 32.2 | 415 | 6 |
| 32.5 | 611 | 8 |
| 38.7 | 292 | 4 |
| 40.2 | 316 | 4 |
| 44.4 | 223 | 3 |
| 45.0 | 164 | 2 |

Those data support the fact that the free base can exist in the form of two different non-solvated polymorphs or a mixture thereof. Therefore, further characterization of those forms has been carried our as follows, separately.

### Hygroscopicity assessment by DVS

The hygroscopic character of the two crystal forms has been investigated as described above.

Upon drying of the crystal form "B" of the free base at 0%RH, a small (0.2%) mass loss is detected. The sample mass uptake of the crystal form "A" is 0.2% at 60%RH and 0.3% at 95%RH. Based on the commonly used criteria (2% maximum uptake at 60%RH), the free base physical form B is to be considered as non-hygroscopic.

### DSC and TGA characterization

The two crystal forms have been characterized as described above. On the temperature interval studied, a single endothermic event is detected by DSC and can be interpreted as the melting of the free base physical form A as follows:
- The onset and peak temperature are respectively 250 and 251°C,
- The melting enthalpy is 79 J/g.

No mass loss occurs during melting as evidenced by the TGA and DSC overlaid traces for sample (1) (Figure 2B).

### Solubility assessment

The solubility of the two crystal forms have been characterized as described below:
A suspension is prepared in the buffer. Magnetic stirring is applied at 100 rpm.

The concentration of the free base in solution in buffer solution at pH 7.4 (38g Na₂HPO₄, 12 H₂O + 3,8g NaH₂PO₄, 2 H₂O completed to 1L with de-ionized water) is determined after 30 min, 1 min, 2h, 4h, 6h and 24h at ambient temperature protected from light. For that purpose, the samples are withdrawn and filtered on filter Millex PVDF 0.22µm (diameter=13mm) and diluted in water/acetonitrile 50/50 (v/v) in order to reach a concentration in the range of standard solutions concentrations. Two separated dilutions are performed for each sample and the final result is the mean of these two dilutions results. The concentration of the solubilized fraction is determined by HPLC by a short gradient reverse phase standard method with UV-diode array detection solution.

The following tables display the solubilized freebase concentrations as a function of time at pH 7.4 (Table 10) at 20°C (samples protected from light) for:
- Form A : crystalline freebase (sample 2),
- Form B : crystalline freebase (sample 1).

**Table 10**

| | Solubilized fraction (mg/mL) | |
|---|---|---|
| Time | Form A | Form B |
| 30min | 0.33 | 0.23 |
| 1h | 0.35 | 0.25 |
| 2h | 0.37 | 0.26 |
| 4h | 0.39 | 0.27 |
| 6h | 0.40 | 0.28 |
| 24 h | 0.51 | 0.26 |
| **Solubility** (mg/mL) | **0.5** | **0.3** |

The solubility data above indicate that the solubility of form B at pH 7.4 is 0.3 mg/mL which is significantly less than 0.5 mg/mL obtained for form. This finding is in agreement with the relative stability conclusions drawn from thermal analysis data (DSC) below: form B, more stable than form A, is also less soluble.

### Thermal analysis data

In Table 11 below, the onset temperatures and melting enthalpies for both free base crystal forms A and B as measured by DSC as described above are reported.

According to the Heat-of-Fusion Rule proposed by Burger and Ramburger [Giron et al., 1995, Thermochim Acta, 248, 1-59)*,* if the higher melting form has the lower heat of fusion, the polymorphic system is usually enantiotropic; otherwise it is monotropic. Therefore, it is possible to conclude that free base polymorph form A and free base polymorph form B are monotropically related, form A being the thermodynamically less stable form whatever the temperature.

**Table 11**

| **Form** | **Melting** | |
|---|---|---|
| | **Onset temperature (°C)** | **Enthalpy (J/ g)** |
| A | 195 | 61 |
| B | 250 | 79 |

Altogether those data support the fact that form B has the following characteristics:
- The material is crystalline and not hygroscopic;
- It has a high melting point (250°C),
- it presents an acceptable solubility profile for oral route over the whole GI tract pH range: 0.3 mg/mL at pH 7.4, more than 30 mg/mL at pH 1.

### Example 3: Preparation of Polymorph Form B by a process of the invention

Based on the advantageous of free base form B, it would be advantageous to develop a method allowing obtaining polymorph form B is an essentially pure form in a reproducible manner.

Example of a method of the invention for obtaining polymorph form B is an essentially pure form is provided below and was applied to the manufacturing of clinical batches on the kilo scale.

A solution of the free base of 2-(2-chlorophenyl)-4-[3-(dimethylamino)phenyl]-5-methyl-1H-pyrazolo[4,3-c] pyridine-3,6(2H,5H)-dione in DCM is provided at a concentration of about 5-15%such as 10%.

This solution might be obtained either by directly dissolving the free base of 2-(2-chlorophenyl)-4-[3-(dimethylamino)phenyl]-5-methyl-1H-pyrazolo[4,3-c] pyridine-3,6(2H,5H)-dione into DCM or by adding 50 mL to an aqueous phase containing a concentration of 10 g of the free base after the last step of synthesis of 2-(2-chlorophenyl)-4-[3-(dimethylamino)phenyl]-5-methyl-1H-pyrazolo[4,3-c] pyridine-3,6(2H,5H)-dione from {1-(2-Chloro-phenyl)-4-[(3-dimethylamino-phenyl)-methylamino-methylene]-5-oxo-4,5-dihydro-1H-pyrazol-3-yl}-acetic acid methyl ester.

### Co-distillatiou of DCM with ethyl acetate

Co-distillation of DCM with ethyl acetate as follows led to polymorph form B in an essentially pure form:

### Step 1: extraction with DCM

50 mL of dichloromethane was added to an 90 mL of an aqueous solution containing circa 10 g of the free base of 2-(2-chlorophenyl)-4-[3-(dimethylamino)phenyl]-5-methyl-1H-pyrazolo[4,3-c] pyridine-3,6(2H,5H)-dione .The pH was adjusted with 32% HC1 (11-13 mL) from ca. 13.5 to a pH of 5-7, preferable to ca. 5.5-6.2. The temperature was maintained < 15°C during pH adjustment.

The phases were separated were the lower organic phase is the product rich phase. The aqueous phase was extracted with 30 mL dichloromethane and the resulting organic phase was combined with the lower organic phase extracted from the previous extraction step.

The combined organic phases were then washed with 30 mL of water and concentrated *in vacuo* to ca 30 mL.

### Step 2: dilution with ethyl acetate

145 mL of ethyl acetate was added to the concentrate and concentration repeated *in vacuo* to ca 30 mL.

The product suspension was then stirred for minimum 1 hour (e.g. overnight stirring). 145 mL of ethyl acetate was further added and the mixture was concentrated *in vacuo* to ca. 30 mL. The product suspension was then stirred over night at 18-25°C.

The resulting suspension is then filtered and the resulting solid product washed with 20 mL of ethyl acetate in portions. It is then dried under vacuum until a loss on drying of not more than 0.5% is reached. The product is obtained as polymorph B.

The resulting product is characterized as being a polymorph form B in essentially pure form, as characterized by a powder X-ray diffraction pattern (PXRD) (Figure 3C) which includes main peaks a 2-Theta angles as reported under table 12 below.

### Co-distillation of methanol with ethyl acetate

A further optimization of the product of the invention using an intermediate step of co-distillation of methanol/ethyl acetate allowed followed by a co-distillation with ethyl acetate allowed to reduce the levels of DCM that are used, to remove methanol and to increase the yields, while achieving a polymorph form B in an essentially pure form. An Example of such process is described below:
The same step 1 as in the process as described above was repeated. Then, step 1 was followed by the following modified step 2:

### Step 2: addition with methanol

145 mL of methanol was added to the mixture which was then concentrated *in vacuo* to circa 50 mL. The dichloromethane to methanol molar ratio was determined by gas chromatography. ***Step 3: extraction with ethyl acetate*** 95 mL of methanol was then added and then 45 mL of ethyl acetate. The mixture was concentrated *in vacuo* to circa 45 mL.

During the distillation or during subsequent stirring, the product starts to crystallize and seeds were added during the distillation.

The product suspension was then stirred for minimum 1 hour (e.g. overnight stirring).

145 mL of ethyl acetate is further added and the mixture concentrated *in vacuo* to circa 45 mL. This step was repeated until the methanol content should be < 5.0%. The product suspension was then stirred over night at 18-25°C (such as for example stirring for 1 or 2 days).

The resulting suspension is then filtered and the resulting solid product washed with 20 mL of ethyl acetate in portions.

The product (8.5 g) was then dried *in vacuo* at T < 50°C and was obtained in a yield of 84% and a purity of 99.5% as determined by HPLC. Characterization of the obtained product confirmed the obtaining of polymorph form B of the free base, in an essentially pure form.

The product (8.5 g) was then dried *in vacuo* at T < 50°C and was obtained in a yield of 83-92 % and a purity of 99.5%. Characterization of the obtained product confirmed the obtaining of polymorph form B of the free base, in an essentially pure form as shown by DSC (Figure 3A) and PXRD measured as described above (Figure 3C) as described below.

The resulting product is characterized as being a polymorph form B in, as characterized by powder X-Ray Diffraction (PXRD) carried out on samples placed into standard glass capillaries (Ø = 0.5 mm) (Figure 3C). The measurements were performed at room temperature with a D8 Bruker Advance Diffractometer (Cu-Kα1 = 1.54059 Å, Johansson primary beam monochromator, position sensitive detector) in transmission mode with rotation of the sample. Data were collected in a two-theta range of 1.5-50°. The tube voltage and current were 40 kV and 40 mA, respectively. The derived theta angles for which intensity is higher than 50 are listed below in Table 12:

**Table 12**

| **Angle** | **Intensity %** |
|---|---|
| **2-Theta (°)** | |
| 9,853 | 71,6 |
| 13,954 | 55,0 |
| 16,110 | 59,4 |
| 17,186 | 51,6 |
| 17,664 | 71,2 |
| 17,838 | 63,4 |
| 18,991 | 100,0 |
| 23,654 | 71,2 |
| 23,833 | 91,4 |
| 25,921 | 60,5 |
| 28,328 | 52,1 |

Based on the spectrum analysis above, it could be derived that this crystalline form is the same as form B identified under Figure 2A (1) as shown in the superposition of Figure 3C.

The resulting product is further characterized as being a polymorph form B by IR spectroscopy as described above and showed absorption bands at 3438 cm-1, 2989 cm-1, 2817 cm-1, 1715 cm-1 1644 cm-1 1602 cm-1 1581 cm-1 1563 cm-1 1500 cm-1 1478 cm-1 1432 cm-1 1358 cm-1 1311 cm-1 1276 cm-1 1230 cm-1 1130 cm-1 994 cm-1, 768 cm-1, 742 cm-1, and 700 cm-1 (Figure 3B).

These data show that when the product is extracted from water with a polar solvent (e.g. DCM), and this polar solvent is progressively replaced first with another polar solvent (e.g. methanol) and then by a non polar solvent (e.g. ethyl acetate, isopropyl acetate), the crystallization process is smooth and leads to good filtration properties of the obtained polymorph. Further, this leads also to an improved yield from 68% to 83-92%.

## Claims

1. A crystalline free base form of 2-(2-chlorophenyl)-4-[3-(dimethylamino)phenyl]-5-methyl-1H-pyrazolo[4,3-c]pyridine-3,6(2H,5H)-dione.

2. A crystalline free base form according to claim 1 comprising a crystalline polymorph form B of 2-(2-chlorophenyl)-4-[3-(dimethylamino)phenyl]-5-methyl-1H-pyrazolo[4,3-c] pyridine-3,6(2H,5H)-dione **characterized by** a powder x-ray diffraction pattern comprising diffraction peaks at 2θ values: 9.9 ± 0.2°, 11.3 ± 0.2°, 17.7 ± 0.2°, 19.0 ± 0.2° and 23.9 ± 0.2°.

3. A crystalline free base form according to claim 2, comprising form B in an essentially pure form.

4. A crystalline free base form according to claim 2 or 3, wherein the crystalline polymorph form B has a DSC curve showing an endothermic peak with an onset temperature of about 250°C.

5. A crystalline free base form according to any one of claims 2 to 4, wherein the crystalline polymorph form B has an infrared spectrum comprising peaks at wavenumbers of 3438 cm-1, 2989 cm-1, 2817 cm-1, 1715 cm-1 1644 cm-1 1602 cm-1 1581 cm-1 1563 cm-1 1500 cm-1 1478 cm-1 1432 cm-1 1358 cm-1 1311 cm-1 1276 cm-1 1230 cm-1 1130 cm-1 994 cm-1, 768 cm-1, 742 cm-1, and 700 cm-1.

6. A crystalline free base form according to claim 1 comprising a crystalline polymorph form A of 2-(2-chlorophenyl)-4-[3-(dimethylamino)phenyl]-5-methyl-1H-pyrazolo[4,3-c] pyridine-3,6(2H,5H)-dione **characterized by** a powder x-ray diffraction pattern comprising diffraction peaks at 2θ values of 9.1 ± 0.2°, 11.4± 0.2°, 13.9 ± 0.2°, 15.8± 0.2°, 16.2 ± 0.2°, 18.7 ± 0.2°, 24.2 ± 0.2° and 26.5 ± 0.2°..

7. A process for preparing crystalline freebase form of any one of the preceding claims comprising the steps of:
(i) Providing a mixture of an aqueous solution of the 2-(2-chlorophenyl)-4-[3-(dimethylamino)phenyl]-5-methyl-1H-pyrazolo[4,3-c]pyridine-3,6(2H,5H)-dione free base in in an apolar solvent or a mixture thereof;
(ii) Adding a polar solvent to the mixture;
(iii) concentrating the mixture *in vacuo* under stirring at a temperature from about a temperature from about 10-50°C;
(iv) isolating the solid product.

8. A process according to claim 7 wherein steps from (ii) and to (iii) further comprise the following steps:
(iia) Adding a polar solvent to the mixture;
(iiia) concentrating the mixture *in vacuo* under stirring at a temperature from about 10-50°C, for example from about 20 to about 25°C;
(iib) progressively adding a non-polar solvent to the mixture during the concentration step for replacing progressively the polar solvent that is evaporating and repeating steps (iiia) to (iib) until the polar solvent is essentially removed from the mixture, for example that its content is not higher than 5 to 10% vol., typically less than about 5% in vol.;
(iiib) completing the final concentration step.

9. A process according to claim 7 or 8 wherein the pH of the solution under step (i) is adjusted in a pH range of 5-7, in particular between about 5.5 and about 6.2.

10. A process according to any one of claims 7 to 9 wherein, during the concentration step (iii), a polar is further added at least once.

11. A process according to any one of claims 7 to 10 wherein the isolation step (iv) comprises a step of filtering the resulting mixture.

12. A process according to any one of claims 7 to 11 wherein the isolation step comprises a step of micronizing the dried the resulting product.

13. A crystalline free base form obtainable by a process of any one of claims 7 to 12.

14. A crystalline free base form according to anyone of claims 1 to 6 and 13 for use as a medicament.

15. A pharmaceutical composition comprising a crystalline free base form according to any one of claims 1 to 6 and 13 and a pharmaceutically acceptable carrier, diluent or excipient.

16. Use of a crystalline free base form of any one of claims 1 to 6 and 13 or a pharmaceutical formulation thereof for the preparation of a pharmaceutical composition for the treatment and/or prophylaxis of a NADPH oxidase related disorder.
